# EUROPÄISCHE PATENTSCHRIFT

(11) **EP 2 869 811 B1**
(45) Veröffentlichungstag und Bekanntmachung des Hinweises auf die Patenterteilung: **10.10.2018**
(21) Anmeldenummer: 13728396.6
(22) Anmeldetag: 13.06.2013
(51) Int. Cl.: A61K 8/73, A61Q 19/06, A61Q 19/08, A61K 8/9789, A61K 31/728, A61K 36/575

(54) **WIRKSTOFFKOMBINATIONEN AUS MAGNOLIENRINDENEXTRAKT UND HYALURONSÄURE UND IHRE KOSMETISCHE UND/ODER DERMATOLOGISCHE VERWENDUNG**
ACTIVE INGREDIENT COMBINATIONS OF MAGNOLIA BARK EXTRACT AND HYALURONIC ACID AND THE COSMETIC AND/OR DERMATOLOGICAL USE THEREOF
ASSOCIATIONS DE PRINCIPES ACTIFS COMPOSÉES D'EXTRAIT D'ÉCORCE DE MAGNOLIA ET D'ACIDE HYALURONIQUE ET LEUR UTILISATION COSMÉTIQUE ET/OU DERMATOLOGIQUE

(30) Priorität: 06.07.2012 DE 102012211807
(43) Veröffentlichungstag der Anmeldung: 13.05.2015
(73) Patentinhaber: Beiersdorf AG, 20253 Hamburg (DE)
(72) Erfinder: ILSEMANN, Corinna, 22453 Hamburg (DE); WINNEFELD, Marc, 22880 Wedel (DE); SCHLÄGER, Torsten, 22297 Hamburg (DE); HOLTZMANN, Ursula, 22309 Hamburg (DE); HIDDEMANN, Sarah, 22523 Hamburg (DE); KURZ, Nadeshda, 20535 Hamburg (DE); RUHS, Joanna, 22043 Hamburg (DE)
(86) Internationale Anmeldenummer: PCT/EP2013/062274
(87) Internationale Veröffentlichungsnummer: WO 2014/005818

(56) Entgegenhaltungen:
- DE-A1-102008 034 265
- DE-A1-102010 015 790
- DE-A1-102010 063 895
- US-A1- 2008 260 869
- "Korres Magnolia Bark Day Cream for First Wrinkles, Tagescreme mit Magnolienrindenextrakt für erste Fältchen 40ml", 20120427 , 27. April 2012 (2012-04-27), Seiten 1-2, XP007922494, Gefunden im Internet: URL:https://web.archive.org/web/2012042723 5134/http://www.greenglam.... 1 of [gefunden am 2014-01-22]
- Anonymous: "DaUnBee Activating Liposome Lotion(120ml) from SkinCure, Inc., Korea", , 28. September 2009 (2009-09-28), XP055098178, Gefunden im Internet: URL:http://www.ec21.com/product-details/Da UnBee-Activating-Liposome-Lotion-120ml--63 95960.html [gefunden am 2014-01-23]

## Beschreibung

Die vorliegende Erfindung betrifft kosmetische bzw. dermatologische Zubereitungen, enthaltend Wirkstoffe zur Pflege und zum Schutze der Haut, insbesondere der empfindlichen Haut wie auch ganz besonders im Vordergrunde stehend der durch intrinsische und/oder extrinsische Faktoren gealterten oder alternden Haut sowie die Verwendung solcher Wirkstoffe und Kombinationen solcher Wirkstoffe auf dem Gebiete der kosmetischen und dermatologischen Hautpflege.

Unter Kosmetik kann man alle Maßnahmen zusammenfassen, die aus ästhetischen Gründen Veränderungen an Haut und Haaren vornehmen oder zur Körperreinigung angewendet werden. Kosmetik bedeutet also, das Körperäußere zu pflegen, zu verbessern und zu verschönern, um auf sichtbare, fühlbare und riechbare Weise sowohl den Mitmenschen als auch sich selbst zu gefallen.

Wird diese Funktion gestört, kann es zu verstärkter Resorption toxischer oder allergener Stoffe oder zum Befall von Mikroorganismen und als Folge zu toxischen oder allergischen Hautreaktionen kommen.

Ziel der Hautpflege ist es ferner, den durch das tägliche Waschen verursachten Fett- und Wasserverlust der Haut auszugleichen. Dies ist gerade dann wichtig, wenn das natürliche Regenerationsvermögen nicht ausreicht. Außerdem sollen Hautpflegeprodukte vor Umwelteinflüssen, insbesondere vor Sonne und Wind, schützen und die Hautalterung verzögern.

Die chronologische Hautalterung wird z.B. durch endogene, genetisch determinierte Faktoren verursacht. In Epidermis und Dermis kommt es alterungsbedingt z.B. zu folgenden Strukturschäden und Funktionsstörungen, die auch unter den Begriff "Senile Xerosis" fallen können:
a) Trockenheit, Rauhigkeit und Ausbildung von Trockenheitsfältchen,
b) Juckreiz und
c) verminderte Rückfettung durch Talgdrüsen (z.B. nach Waschen).

Exogene Faktoren, wie UV-Licht und chemische Noxen, können kumulativ wirksam sein und z.B. die endogenen Alterungsprozesse beschleunigen bzw. sie ergänzen. In Epidermis und Dermis kommt es insbesondere durch exogene Faktoren z.B. zu folgenden Strukturschäden- und Funktionsstörungen in der Haut, die über Maß und Qualität der Schäden bei chronologischer Alterung hinausgehen:
d) Sichtbare Gefäßerweiterungen (Teleangiektasien, Cuperosis);
e) Schlaffheit, Volumenverlust und Ausbildung von Falten;
f) lokale Hyper-, Hypo- und Fehlpigmentierungen (z.B. Altersflecken) und
g) vergrößerte Anfälligkeit gegenüber mechanischem Stress (z.B. Rissigkeit).

Die vorliegende Erfindung betrifft insbesondere Produkte zur Pflege der auf natürliche Weise gealterten Haut, sowie der unter a), e) und g) aufgeführten Phänomene.

Produkte zur Pflege erschlaffter, insbesondere gealterter Haut sind an sich bekannt. Sie enthalten z.B. Retinoide (Vitamin A-Säure und/oder deren Derivate) bzw. Vitamin A und/oder dessen Derivate. Ihre Wirkung auf die Strukturschäden ist allerdings umfangsmäßig begrenzt. Darüber hinaus gibt es bei der Produktentwicklung erhebliche Schwierigkeiten, die Wirkstoffe in ausreichendem Maße gegen oxidativen Zerfall zu stabilisieren. Die Verwendung Vitamin A-Säurehaltiger Produkte bedingt darüber hinaus oft starke erythematöse Hautreizungen. Retinoide sind daher nur in geringen Konzentrationen einsetzbar.

Oftmals wird mit der erschlafften Haut auch eine Begleiterscheinung der Übergewichtigkeit und/oder der damit häufig einhergehenden sogenannten Cellullite verbunden.

Das Körperbewußtsein der Verbraucher ist in den vergangenen Jahren deutlich gestiegen. Dabei werden neben reinigenden und pflegenden Anwendungen auch zunehmend Maßnahmen ergriffen, um die Körpersilhouette zu verbessern. Die Cellulite - ein weit verbreitetes Phänomen - nimmt dabei eine zentrale Stellung ein. Das sichtbare Bild der Cellulite beruht auf einer Zunahme von Fettpolstern in der Subcutis (Unterhautfettgewebe), einer Bindegewebsschwäche sowie einer Minderung der Durchströmungsverhältnisse in den Blut- und Lymphbahnen. Die Ursache ist somit eine zum Teil anlagebedingte Schwächung des Bindegewebes mit gleichzeitigem Auftreten von vergrößerten Fettzellkammern infolge von Übergewicht, unausgewogener Ernährung, Bewegungsmangel. Die Entstehung der Cellulite kann ferner auf eine erhöhte Durchlässigkeit der Haargefäßwände zurückgeführt werden, die das Eindringen von Wasser ins Bindegewebe erlaubt.
Daneben kann an den betroffenen Hautbereichen ein lokaler Mangel an Testosteron bestehen. Jedenfalls ist Cellulite eine Erscheinung die fast niemals bei Männern zu beobachten ist. Aufgabe der vorliegenden Erfindung war es somit, Wege zu finden, die die Nachteile des Standes der Technik vermeiden. Insbesondere war es eine Aufgabe der vorliegenden Erfindung, Zubereitungen zur Verfügung zu stellen, die eine vorteilhafte Straffung erschlaffter Haut bewirken können.
Es hat sich überraschenderweise herausgestellt, daß Wirkstoffkombinationen aus Magnolienrindenextrakt und Hyaluronsäure gemäß Anspruch 1 sowie die Verwendung von Wirkstoffkombinationen aus Magnolienrindenextrakt und Hyaluronsäure gemäß Anspruch 1 zur Straffung und/oder Festigung der Haut, insbesondere in kosmetischen oder dermatologischen Zubereitungen den Nachteilen des Standes der Technik abhilft.
Die Verwendung erfindungsgemäßer Wirkstoffkombinationen bzw. kosmetische oder dermatologische Zubereitungen mit einem wirksamen Gehalt an solchen Wirkstoffkombinationen z. B. in Form der weiter unten aufgeführten Beispiele - führt in überraschender Weise zu einer erheblichen Verbesserung des Aussehens der Haut.
Durch die Verwendung erfindungsgemäßer Zubereitungen wird ferner die Hautfeuchtigkeit gesteigert und die Lipid-Synthese angeregt und damit das Hautvolumen positiv beeinflusst.
Es war überraschend, daß durch Anwenden erfindungsgemäßer Wirkstoffkombinationen bzw. kosmetische oder dermatologische Zubereitungen mit einem wirksamen Gehalt an solchen gemäß Anspruch 1 die Faltentiefe und Faltenanzahl intrinsisch oder extrinsisch gealterter Haut vermindert und somit. das Erscheinungsbild der Altershaut verbessert wird.
Es war ferner überraschend, daß durch Anwenden erfindungsgemäßer Wirkstoffkombinationen bzw. kosmetische oder dermatologische Zubereitungen mit einem wirksamen Gehalt an solchen Wirkstoffkombinationen die Elastizität der Haut, und daher Cellulite bzw. das Erscheinungsbild der sog. "Orangenhaut" verbessert wird.

Magnolien (Magnolia) sind eine Pflanzengattung in der Familie der Magnoliengewächse (Magnoliaceae). Diese enthält etwa 230 Arten, die aus Ostasien und Amerika stammen. Erfindungsgemäß kann der Magnolienrindenextrakt grundsätzlich allen Arten der Gattung Magnolia entnommen werden, wobei allerdings Magnolia grandiflora, und insbesondere Magnolia officinalis bevorzugt werden.

Magnolien-Rinde wird in der Traditionellen Chinesischen Medizin (TCM) vor allem bei "Stagnation of qi" (low energy), emotionalem Stress, Entzündungen, innerer Unruhe und Angstzuständen eingesetzt.

Die Herstellung des verwendeten Extraktes erfolgt mit Hilfe von superkritischem CO₂ oder Ethanol oder einer Ethanol / Wasser Mischung.

Magnolol ist durch folgende Struktur gekennzeichnet:

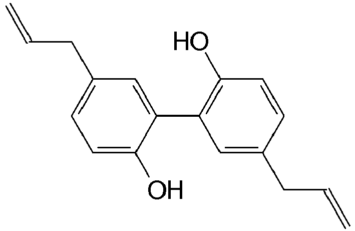

Honokiol ist durch folgende Struktur gekennzeichnet:

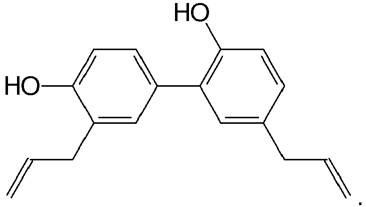

Die erfindungsgemäßen kosmetischen oder dermatologischen Zubereitungen können wie üblich zusammengesetzt sein und zur Behandlung, der Pflege und der Reinigung der Haut und/oder der Haare und als Schminkprodukt in der dekorativen Kosmetik dienen. Sie enthalten bevorzugt 0,001 Gew.-% bis 10 Gew.-%, bevorzugt 0,05 Gew.-% bis 5 Gew.-%, insbesondere 0,1 - 2,0 Gew.-%, bezogen auf das Gesamtgewicht der Zubereitungen, an Magnolienrindenextrakt.

Hyaluronsäure ist ein Polysaccharidderivat, welches durch folgendes Strukturelement gekennzeichnet ist:

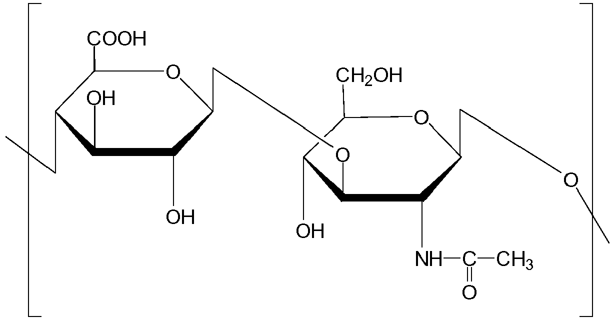

Hyaluronsäure ist ein Glycosaminoglycan, das im Bindegewebe, im Glaskörper, in der Nabelschnur und in der Synovialflüssigkeit der Gelenke vorkommt. Hyaluronsäure ist in besonders hohem Maße in frühen Embryonalstadien vorhanden und wirkt hier an Zellwanderungen mit, indem sie durch ihre lockere Struktur und einen hohen Wassergehalt die sie auf ihrer Oberfläche exprimierenden Zellen gegeneinander abschirmt und so deren freie Beweglichkeit ermöglicht. Hyaluronsäure ist an der Wundheilung beteiligt

Hyaluronsäure ist eine hochmolekulare Verbindung mit Molekulargewichten zwischen 40000 und mehreren Millionen. Es ist im Sinne der vorliegenden Erfindung vorteilhaft, wenn die eingesetzte Hyaluronsäure ein mittleres Molmassenmaximum zwischen 40 kDa und 60 kDa und/oder 1000 kDa und 2200 kDa aufweist. Es ist erfindungsgemäß gleichermaßen vorteilhaft, wenn die Hyaluronsäure in protonierter Form vorliegt, als Salz, als teilprotoniertes Salz, in Lösung dissoziiert oder teildissoziiert.

Erfindungsgemäß beträgt die Konzentration an Hyaluronsäure bezogen auf das Gesamtgewicht der Zubereitung vorteilhaft 0,001 bis 10 Gewichts-%, bevorzugt von 0,05 bis 5 Gewichts-% und ganz besonders bevorzugt von 0,01 bis 2 Gewichts-%, jeweils bezogen auf das Gesamtgewicht der Zubereitung.

Vorteilhaft werden Gewichtsverhältnisse von Magnolienrindenextrakt und Hyaluronsäure aus den Bereichen 10 : 1 bis 1 : 1, bevorzugt von 8 : 1 bis 2 : 1, besonders bevorzugt von 6 : 1 bis 3 : 1, gewählt.

Erfindungsgemäß können Zubereitungen, welche die erfindungsgemäßen Wirkstoffkombinationen enthalten, übliche Antioxidantien eingesetzt werden.

Vorteilhaft werden die Antioxidantien gewählt aus der Gruppe bestehend aus Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Carotinoide, Carotine (z.B. α-Carotin, β-Carotin, Lycopin) und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathioninsulfoximin) in sehr geringen verträglichen Dosierungen (z.B. pmol bis µmol/kg), ferner (Metall)-Chelatoren (z.B. α-Hydroxyfettsäuren, Palmitinsäure, Phytinsäure, Lactoferrin), α-Hydroxysäuren (z.B. Citronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Alanindiessigsäure, Flavonoide, Polyphenole, Catechine, Vitamin C und Derivate (z.B. Ascorbylpalmitat, Mg-Ascorbylphosphat, Ascorbylacetat), Tocopherole und Derivate (z.B. Vitamin-E-acetat), sowie Koniferylbenzoat des Benzoeharzes, Rutinsäure und deren Derivate, Ferulasäure und deren Derivate, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Zink und dessen Derivate (z.B. ZnO, ZnSO₄) Selen und dessen Derivate (z.B. Selenmethionin), Stilbene und deren Derivate (z.B. Stilbenoxid, Trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Die Menge der Antioxidantien (eine oder mehrere Verbindungen) in den Zubereitungen beträgt vorzugsweise 0,001 bis 30 Gew.-%, besonders bevorzugt 0,05 - 20 Gew.-%, insbesondere 1 - 10 Gew.-%, bezogen auf das Gesamtgewicht der Zubereitung.

Besonders vorteilhafte Verkörperungen der vorliegenden Erfindung bestehen in Wirkstoffkombinationen aus Magnolienrindenextrakt, Hyaluronsäure und einem Anisfruchtextrakt sowie die Verwendung von Wirkstoffkombinationen aus Magnolienrindenextrakt, Hyaluronsäure und einem Anisfruchtextrakt zur Straffung und/oder Festigung der Haut, insbesondere in kosmetischen oder dermatologischen Zubereitungen.

Es ist erfindungsgemäß insbesondere vorteilhaft, einen wäßrigen Frucht-Extrakt aus Anis (INCI: Pimpinella anis fruit extract), der reich an anorganischen Mineralien wie Natrium- und Magnesium-Ionen, besonders an Kalium-Ionen ist und mit Butylenglycol 0,36% und Parabenen 0,14% konserviert ist und durch enzymatische Hydrolyse in Wasser solubilisierter Anis-Früchte erhältlich ist, wobei das Verhältnis von Rohmaterial zu Extrakt etwa 1 : 2 beträgt und unter dem Handelsnamen Bioxilift ® bei der Gesellschaft SILAB, (Brive Cedex, France) erhältlich ist.

Bei Anisfruchtextrakt (Bioxylift ®, Fa. SILAB) handelt es sich um einen wäßrigen Frucht-Extrakt aus Anis.

Der Extrakt weist eine bernsteinartige Farbe auf und hat einen charakteristischen Anis-Geruch. Er ist reich an anorganischen Mineralien wie Natrium- und Magnesiumionen, besonders aber an Kaliumionen.

Die spezifische Zusammensetzung beträgt:

| | |
|---|---|
| Trockenmasse: | 40 - 60 g/l |
| Mineralasche: | 11 - 18 g/l |
| Gesamtprotein: | 12 - 20 g/l |
| pH: | 4,5 - 5,5 |

Der Extrakt ist unkonserviert oder auch konserviert herstell- und lagerbar. Seine Herstellung wird in der Internationalen Patentanmeldung WO 02/102347 beschrieben.

Bevorzugt enthalten kosmetische oder dermatologische Zubereitungen gemäß der Erfindung 0,001 bis 30 Gew.-%, bevorzugt 0,1 bis 15 Gew.-%, besonders bevorzugt 0,5 bis 7 Gew.-% an, jeweils bezogen auf das Gesamtgewicht der Zubereitung.

Erfindungsgemäß besonders bevorzugt werden Gewichtsverhältnis aus
a) Magnolienrindenextrakt
b) Hyaluronsäure und
c) Anisfruchtextrakt
wie a : b : c gewählt, wobei a, b und c unabhängig voneinander rationale Zahlen von 1 bis 50 , bevorzugt von 1 bis 40 darstellen können.

Insbesondere bevorzugt für Hyaluronsäure mit einem mittlerem Molmassenmaximum zwischen 1000 kDa und 2200 kDa ist ein Gewichtsverhältnis von etwa 5 : 1 : 30 bis 3 : 1 zu 26.
Für Hyaluronsäure mit einem mittlerem Molmassenmaximum zwischen 40 kDa und 60 kDa ist ein Gewichtsverhältnis von etwa 8 : 1 : 45 bis 3 : 1 : 26 bevorzugt.

Die Prophylaxe bzw. die kosmetische oder dermatologische Behandlung mit dem erfindungsgemäß verwendeten Wirkstoff bzw. mit den kosmetischen oder topischen dermatologischen Zubereitungen mit einem wirksamen Gehalt an erfindungsgemäß verwendetem Wirkstoff erfolgt in der üblichen Weise, und zwar dergestalt, daß der erfindungsgemäß verwendete Wirkstoff bzw. die kosmetischen oder topischen dermatologischen Zubereitungen mit einem wirksamen Gehalt an erfindungsgemäß verwendetem Wirkstoff auf die betroffenen Hautstellen aufgetragen wird.

Erfindungsgemäße Emulsionen im Sinne der vorliegenden Erfindung, z.B. in Form einer Creme, einer Lotion, einer kosmetischen Milch sind vorteilhaft und enthalten z.B. Fette, Öle, Wachse und/oder andere Fettkörper, sowie Wasser und einen oder mehrere Emulgatoren, wie sie üblicherweise für einen solchen Typ der Formulierung verwendet werden.

Es ist auch möglich und vorteilhaft im Sinne der vorliegenden Erfindung, den erfindungsgemäß verwendeten Wirkstoff in wäßrige Systeme bzw. Tensidzubereitungen zur Reinigung der Haut und der Haare einzufügen.

Es ist dem Fachmanne natürlich bekannt, daß anspruchsvolle kosmetische Zusammensetzungen zumeist nicht ohne die üblichen Hilfs- und Zusatzstoffe denkbar sind. Darunter zählen beispielsweise Konsistenzgeber, Füllstoffe, Parfüm, Farbstoffe, Emulgatoren, zusätzliche Wirkstoffe wie Vitamine oder Proteine, Lichtschutzmittel, Stabilisatoren, Insektenrepellentien, Alkohol, Wasser, Salze, antimikrobiell, proteolytisch oder keratolytisch wirksame Substanzen usw.

Mutatis mutandis gelten entsprechende Anforderungen an die Formulierung medizinischer Zubereitungen.

Medizinische topische Zusammensetzungen im Sinne der vorliegenden Erfindung enthalten in der Regel ein oder mehrere Medikamente in wirksamer Konzentration. Der Einfachheit halber wird zur sauberen Unterscheidung zwischen kosmetischer und medizinischer Anwendung und entsprechenden Produkten auf die gesetzlichen Bestimmungen der Bundesrepublik Deutschland verwiesen (z.B. Kosmetikverordnung, Lebensmittel- und Arzneimittelgesetz).

Es ist dabei ebenfalls von Vorteil, den erfindungsgemäß verwendeten Wirkstoff als Zusatzstoff zu Zubereitungen zu geben, die bereits andere Wirkstoffe für andere Zwecke enthalten.

Die erfindungsgemäßen kosmetischen und/oder dermatologischen Formulierungen können wie üblich zusammengesetzt sein und zur Behandlung der Haut und/oder der Haare im Sinne einer dermatologischen Behandlung oder einer Behandlung im Sinne der pflegenden Kosmetik dienen. Sie können aber auch in Schminkprodukten in der dekorativen Kosmetik eingesetzt werden oder in den kosmetischen und dermatologischen Reinigungsprodukten.

Sofern die kosmetische oder dermatologische Zubereitung eine Lösung oder Lotion darstellt, können als Lösungsmittel verwendet werden: Wasser oder wäßrige Lösungen, ferner Öle, wie Triglyceride der Caprin- oder der Caprylsäure, vorzugsweise aber Rizinusöl, Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren, aber auch Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte.

Insbesondere werden Gemische der vorstehend genannten Lösungsmittel verwendet, so wie bei alkoholischen Lösungsmitteln Wasser ein vorteilhafter weiterer Bestandteil sein kann.

Kosmetische und dermatologische Zubereitungen gemäß der Erfindung, auch z.B. zum Schutze der Haut vor UV-Strahlen, können in verschiedenen Formen vorliegen, wie sie z.B. üblicherweise für diesen Typ von Zubereitungen eingesetzt werden. So können sie z.B. eine Lösung, eine Emulsion vom Typ Wasser-in-ÖI (W/O) oder vom Typ Öl-in-Wasser (O/W), oder eine multiple Emulsionen, beispielsweise vom Typ Wasser-in-ÖI-in-Wasser (W/O/W), Öl-in-Wasser-in-ÖI (O/W/O), ein Gel, eine Hydrodispersion, eine lamellare Phase, eine flüssige isotrope Lösungsphase, eine micellare Phase, eine solide oder dispergierte ein- oder mehrfach hexagonale Phase, eine solide oder dispergierte ein- oder mehrfach kubische Phase, eine lyotrope Phase, eine kristalline Phase, einen festen Stift oder auch ein Aerosol darstellen.

Die erfindungsgemäßen Wirkstoffe können auch besonders vorteilhaft in Mikroemulsionen, beispielsweise wie in der deutschen Offenlegungsschrift DE-195 9 079 beschrieben, verwendet werden.

Die Ölphase der erfindungsgemäßen Zubereitungen wird vorteilhaft gewählt aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen, aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Oleyloleat, Oleylerucat, Erucyloleat, Erucylerucat sowie synthetische, halbsynthetische und natürliche Gemische solcher Ester, z.B. Jojobaöl.

Ferner kann die Ölphase vorteilhaft gewählt werden aus der Gruppe der verzweigten und unverzweigten Kohlenwasserstoffe und -wachse, der Silkonöle, der Dialkylether, der Gruppe der gesättigten oder ungesättigten, verzweigten oder unverzweigten Alkohole, sowie der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 - 18 C-Atomen. Die Fettsäuretriglyceride können beispielsweise vorteilhaft gewählt werden aus der Gruppe der synthetischen, halbsynthetischen und natürlichen Öle, z.B. Olivenöl, Sonnenblumenöl, Sojaöl, Erdnußöl, Rapsöl, Mandelöl, Palmöl, Kokosöl, Palmkernöl und dergleichen mehr.

Auch beliebige Abmischungen solcher Öl- und Wachskomponenten sind vorteilhaft im Sinne der vorliegenden Erfindung einzusetzen.

Vorteilhaft wird die Ölphase gewählt aus der Gruppe 2-Ethylhexylisostearat, Octyldodecanol, Isotridecylisononanoat, Isoeicosan, 2-Ethylhexylcocoat, C₁₂₋₁₅-Alkylbenzoat, Capryl-Caprinsäure-triglycerid, Dicaprylylether.

Besonders vorteilhaft sind Mischungen aus C₁₂₋₁₅-Alkylbenzoat und 2-Ethylhexylisostearat, Mischungen aus C₁₂₋₁₅-Alkylbenzoat und Isotridecylisononanoat sowie Mischungen aus C₁₂₋₁₅-Alkylbenzoat, 2-Ethylhexylisostearat und Isotridecylisononanoat.

Von den Kohlenwasserstoffen sind Paraffinöl, Squalan und Squalen vorteilhaft im Sinne der vorliegenden Erfindung zu verwenden.

Vorteilhaft kann die Ölphase ferner einen Gehalt an cyclischen oder linearen Silikonölen aufweisen oder vollständig aus solchen Ölen bestehen, wobei allerdings bevorzugt wird, außer dem Silikonöl oder den Silikonölen einen zusätzlichen Gehalt an anderen Ölphasenkomponenten zu verwenden.

Vorteilhaft wird Cyclomethicon (Octamethylcyclotetrasiloxan) als erfindungsgemäß zu verwendendes Silikonöl eingesetzt. Aber auch andere Silikonöle sind vorteilhaft im Sinne der vorliegenden Erfindung zu verwenden, beispielsweise Hexamethylcyclotrisiloxan, Polydimethylsiloxan, Poly(methylphenylsiloxan).

Besonders vorteilhaft sind ferner Mischungen aus Cyclomethicon und Isotridecylisononanoat, aus Cyclomethicon und 2-Ethylhexylisostearat.

Vorteilhaft beträgt der Gehalt an der Ölphase zwischen 1 und 50 Gew.-%, bezogen auf das Gesamtgewicht der Zubereitungen, bevorzugt 2,5 - 30 Gew.-%, insbesondere bevorzugt 5 - 15 Gew.-%.

Zur Anwendung werden die erfindungsgemäßen kosmetischen und/oder dermatologischen Formulierungen in der für Kosmetika und Dermatika üblichen Weise auf die Haut in ausreichender Menge aufgebracht.

### Wirknachweis:

### Induktion der Adipogenese

Subkutane, aus dem Oberschenkelbereich unterschiedlicher Spender (LOT L041806, L080608) isolierte, humane Präadipozyten wurden von Zen-Bio Inc. (Research Triangle Park, NC) kommerziell erworben.

Die Kultivierung der Zellen erfolgte in Basalmedium (10% fötales Kälberserum (FCS), 2 mM L-Glutamin, 100 U/mL Penicillin und 100 µg/mL Streptomycin; Cambrex, Verviers, Belgien) für 7 Tage bei 37 °C und 5% CO₂ im Zellinkubator. Das Zellkulturmedium wurde am ersten und am vierten Tag nach der Aussaat erneuert.

Nachfolgend wurden die Zellen in 96 Well-Platten passagiert (1x10⁴ Zellen pro Well). 24 Stunden nach Passage in die 96 Well-Platten wurden die Zellen mit Differenzierungsmedium ohne Indomethacin (Basalmedium mit 10% fötalem Kälberserum (FCS), 2 mM L-Glutamin, 100 U/mL Penicillin und 100 µg/mL Streptomycin, 10 µg/mL Insulin, 1 µM Dexamethason und 500 µM Isobutylmethylxanthin; Cambrex, Verviers, Belgien) mit folgenden Wirkstoffen und Wirkstoffkombinationen inkubiert: Magnolienrindenextrakt (10 µM), Hyaluronsäure (LMW, 1%), Magnolienrindenextrakt (10 µM) + Hyaluronsäure (LMW, 1%).

Als "Negativkontrolle" diente eine Zellpopulation die ohne Wirkstoffzugabe kultiviert wurde. Als "Positivkontrolle" wurde Rosiglitazon (2,25 µM) eingesetzt. Die Färbung der Triglycerid-Akkumulation während der Differenzierung wurde am 7. Tag nach der Induktion mittels AdipoRed Reagenz (Cambrex, Verviers, Belgien) gemäß den Herstellerangaben durchgeführt. Die Fluoreszenz (ex 485 nm, em 572 nm) wurde abschließend mit einem Infinite M200 Platten-Reader (Tecan, Crailsheim, Deutschland) bestimmt.

Die folgenden Beispiele sollen die Verkörperungen der vorliegenden Erfindungen verdeutlichen.

| INCI | Beispiel 1 | Beispiel 2 | Beispiel 3 | Beispiel 4 | Beispiel 5 |
|---|---|---|---|---|---|
| 1,2-Hexanediol | 0,50 | | 0,50 | 0,70 | 0,60 |
| Acrylat/C10-30 Alkylacrylatcrosspolymer | 0,10 | 0,20 | | 0,15 | 0,10 |
| Bis-Ethylhexyloxyphenolmethoxyphenyltriazin | 1,00 | 1,50 | 2,00 | 0,50 | |
| Butylenglycoldicaprylat/dicaprat | | | 2,50 | 1,00 | |
| Butylmethoxydibenzoylmethan | 4,00 | 2,00 | | 3,00 | |
| Butyrospermum Parkii (Shea Butter) | 6,00 | 7,00 | 1,00 | 3,00 | |
| C12-15 Alkyl Benzoat | 2,00 | 1,00 | | 1,00 | |
| Capryl-/Caprinsäuretriglycerid | | | | 3,00 | 4,00 |
| Carbomer | 0,30 | 0,10 | 0,40 | 0,20 | 0,10 |
| Cetearylalkohol | 1,00 | 2,50 | 2,00 | 3,00 | |
| Cetylalkohol | 1,00 | 1,00 | | | 1,50 |
| Cetylpalmitat | | | 2,50 | 2,00 | 4,00 |
| Dibutyladipat | 3,00 | 3,00 | | | |
| Diethylaminohydroxybenzoylhexylbenzoat | | | 3,50 | | |
| Dimethicon | | 0,50 | 0,80 | | 0,30 |
| Ethylhexylglycerin | | | 0,25 | 0,30 | 0,50 |
| Ethylhexylsalicylat | 4,50 | 5,00 | | 3,00 | |
| Ethylhexyltriazin | | | 2,50 | | |
| Glycerin | 10,00 | 9,00 | 8,00 | 12,00 | 8,00 |
| Glycerylstearat | 2,50 | 2,00 | | 2,80 | |
| Glycerylstearatcitrat | | | 1,50 | | 2,50 |
| hydrierte Kokosglyceride | 1,00 | 2,00 | 2,00 | 1,00 | 3,50 |
| Isopropylpalmitat | | | | | 2,50 |
| Magnolia Officinalis Bark Extract | 0,50 | 0,50 | 0,40 | 0,80 | 0,70 |
| Methylparaben | | 0,20 | | | |
| Methylpropanediol | 2,00 | 1,50 | | 2,00 | 1,00 |
| Natriumhyaluronat, mittleres Molmassenmaximum zwischen 1000 kDa und 2200 kDa | 0,15 | | 0,10 | | 0,20 |
| Natriumhyaluronat, mittleres Molmassenmaximum zwischen 40 kDa und 60 kDa | | 0,10 | | 0,15 | |
| Octyldodecanol | | | 2,00 | 2,00 | 3,00 |
| Panthenol | | 2,00 | | | 5,00 |
| Parfum | q.s. | q.s. | q.s. | q.s. | q.s. |
| Phenoxyethanol | 0,60 | 0,60 | 0,80 | 0,40 | 0,80 |
| Phenylbenzimidazolsulfonsäure | 2,00 | 1,00 | | 0,50 | |
| Polymethylsilsesquioxan | | | 2,00 | 1,00 | 1,00 |
| Sodium Stearoyl Glutamate + Natriumchlorid | 0,20 | 0,50 | | 0,10 | |
| Stearylalkohol | | | 1,00 | | 2,00 |
| Synthetisches Bienenwachs | 1,00 | | 2,00 | 2,00 | 3,00 |
| Wasser | ad 100% | ad 100% | ad 100% | ad 100% | ad 100% |
| Wasser + Pimpinella Anisum (Anisfruchtextrakt) | 4,00 | 3,50 | 2,80 | 5,00 | 5,50 |
| Wasser + Natriumhydroxid | 1,10 | 0,90 | 0,40 | 0,70 | 0,10 |
| Wasser + Trinatrium EDTA | 1,20 | 1,00 | 1,10 | 0,80 | 1,00 |

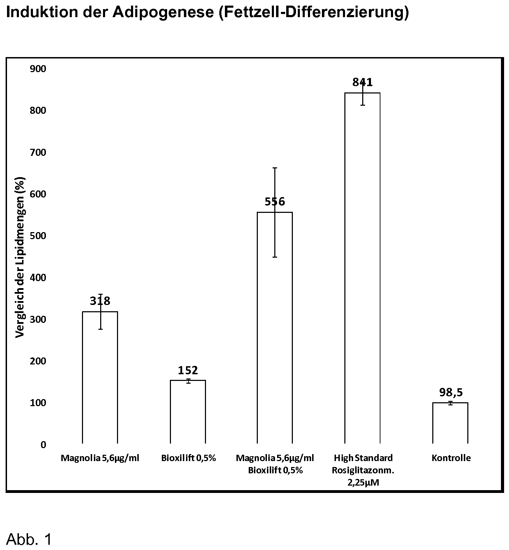

## Patentansprüche

1. Wirkstoffkombinationen aus Magnolienrindenextrakt gewonnen mit Hilfe von superkritischem CO₂ oder Ethanol oder einer Ethanol / Wasser-Mischung aus der Rinde von Magnolia officinalis und Hyaluronsäure, welche ein mittleres Molmassenmaximum zwischen 40 kg/Mol und 60 kg/Mol und/oder 1000 kg/Mol und 2200 kg/Mol aufweist.

2. Wirkstoffkombinationen nach Anspruch1, bei welchen Gewichtsverhältnisse von Magnolienrindenextrakt und Hyaluronsäure aus den Bereichen 10 : 1 bis 1 : 1, bevorzugt von 8 : 1 bis 2 : 1, besonders bevorzugt von 6 : 1 bis 3 : 1, gewählt werden.

3. Kosmetische oder dermatologische Zubereitungen, enthaltend Wirkstoffkombinationen nach einem der vorstehenden Ansprüche.

4. Zubereitungen nach Anspruch 3, enthaltend 0,001 Gew.-% bis 10 Gew.-%, bevorzugt 0,05 Gew.-% bis 5 Gew.-%, insbesondere 0,1 - 2,0 Gew.-%, bezogen auf das Gesamtgewicht der Zubereitungen, an Magnolienrindenextrakt.

5. Zubereitungen nach Anspruch 3 oder 4, enthaltend 0,001 bis 10 Gewichts-%, bevorzugt von 0,05 bis 5 Gewichts-% und ganz besonders bevorzugt von 0,01 bis 2 Gewichts-% an Hyaluronsäure, jeweils bezogen auf das Gesamtgewicht der Zubereitung.

6. Verwendung von Wirkstoffkombinationen nach einem der Ansprüche 1 - 2 oder kosmetischen oder dermatologischen Zubereitungen nach einem der Ansprüche 3 - 5 zur kosmetischen und/oder dermatologischen Behandlung und/oder Prophylaxe der Cellulite.

7. Verwendung von Wirkstoffkombinationen nach einem der Ansprüche 1 - 2 oder kosmetischen oder dermatologischen Zubereitungen nach einem der Ansprüche 3 - 5 zur kosmetischen und/oder dermatologischen Behandlung und/oder Prophylaxe des Erscheinungsbildes der Altershaut.

## Claims

1. Active ingredient combinations of magnolia bark extract, obtained with the aid of supercritical CO₂ or ethanol or an ethanol/water mixture from the bark of Magnolia officinalis, and hyaluronic acid, which has an average maximum molar mass between 40 kg/mol and 60 kg/mol and/or 1000 kg/mol and 2200 kg/mol.

2. Active ingredient combinations according to Claim 1, in which ratios by weight of magnolia bark extract and hyaluronic acid are selected from the ranges from 10:1 to 1:1, preferably from 8:1 to 2:1, particularly preferably from 6:1 to 3:1.

3. Cosmetic or dermatological preparations comprising active ingredient combinations according to either of the preceding claims.

4. Preparations according to Claim 3 comprising 0.001% by weight to 10% by weight, preferably 0.05% by weight to 5% by weight, especially 0.1 - 2.0% by weight, based on the total weight of the preparations, of magnolia bark extract.

5. Preparations according to Claim 3 or 4 comprising 0.001 to 10% by weight, preferably 0.05 to 5% by weight and especially preferably 0.01 to 2% by weight of hyaluronic acid, based in each case on the total weight of the preparation.

6. Use of active ingredient combinations according to either of Claims 1 - 2 or cosmetic or dermatological preparations according to any of Claims 3 - 5 for the cosmetic and/or dermatological treatment and/or prophylaxis of cellulite.

7. Use of active ingredient combinations according to either of Claims 1 - 2 or cosmetic or dermatological preparations according to any of Claims 3 - 5 for the cosmetic and/or dermatological treatment and/or prophylaxis of the appearance of aged skin.

## Revendications

1. Combinaisons d'agents actifs constituées par un extrait d'écorce de magnolia, obtenu à l'aide de CO₂ supercritique ou d'éthanol ou d'un mélange éthanol/eau à partir de l'écorce de Magnolia officinalis, et de l'acide hyaluronique, qui présente un maximum de masse molaire moyenne compris entre 40 kg/mol et 60 kg/mol et/ou entre 1 000 kg/mol et 2 200 kg/mol.

2. Combinaisons d'agents actifs selon la revendication 1, dans lesquelles les rapports en poids entre l'extrait d'écorce de magnolia et l'acide hyaluronique sont choisis dans la plage allant de 10:1 à 1:1, de préférence de 8:1 à 2:1, de manière particulièrement préférée de 6:1 à 3:1.

3. Préparations cosmétiques ou dermatologiques, contenant des combinaisons d'agents actifs selon l'une quelconque des revendications précédentes.

4. Préparations selon la revendication 3, contenant 0,001 % en poids à 10 % en poids, de préférence 0,05 % en poids à 5 % en poids, notamment 0,1 à 2,0 % en poids, par rapport au poids total des préparations, d'extrait d'écorce de magnolia.

5. Préparations selon la revendication 3 ou 4, contenant 0,001 à 10 % en poids, de préférence 0,05 à 5 % en poids et de manière tout particulièrement préférée 0,01 à 2 % en poids d'acide hyaluronique, à chaque fois par rapport au poids total de la préparation.

6. Utilisation de combinaisons d'agents actifs selon l'une quelconque des revendications 1 à 2 ou de préparations cosmétiques ou dermatologiques selon l'une quelconque des revendications 3 à 5 pour le traitement et/ou la prophylaxie cosmétique et/ou dermatologique de la cellulite.

7. Utilisation de combinaisons d'agents actifs selon l'une quelconque des revendications 1 à 2 ou de préparations cosmétiques ou dermatologiques selon l'une quelconque des revendications 3 à 5 pour le traitement et/ou la prophylaxie cosmétique et/ou dermatologique de l'apparence de la peau mûre.
